Europäisches Patentamt

European Patent Office

Office européen des brevets

(11) Publication number: **0 342 028**
**A2**

# (12) EUROPEAN PATENT APPLICATION

(21) Application number: **89304771.2**

(22) Date of filing: **11.05.89**

(51) Int. Cl.⁴: **A 61 B 10/00**

(30) Priority: **12.05.88 US 193415**

(43) Date of publication of application:
**15.11.89 Bulletin 89/46**

(84) Designated Contracting States:
**AT BE CH DE ES FR GB GR IT LI LU NL SE**

(71) Applicant: **ENDOTHERAPEUTICS**
**191 Jefferson Drive**
**Menlo Park, CA 94025 (US)**

(72) Inventor: **Van Buskirk, Robert Scott**
**47 Ramona Road**
**Danville California 94526 (US)**

**Clappier, Robert R.**
**476 Mundell Way**
**Los Altos California 94022 (US)**

(74) Representative: **Harrison, David Christopher et al**
**MEWBURN ELLIS & CO 2/3 Cursitor Street**
**London EC4A 1BQ (GB)**

(54) Urine collection monitor.

(57) An apparatus and method for flow analysis during collection of a urine sample are disclosed, and for simultaneously maintaining the sterile urine sample for use in clinical and microbiological analysis. A disposable vessel for collecting the urine is mounted on a commode adapter, and includes a variable capacitor, with a capacitance value which varies with the amount of urine (or other fluid) in the vessel. The variable capacitor forms a part of a capacitance bridge, which is used to produce a potential relating to the varying capacitance value. A microprocessor controlled by a clock is used to periodically sample the varying capacitance value as fluid flows into the vessel, and thereby determine the flow rates of the fluid and other useful values. A tube is provided which communicates with the interior of the vessel for drawing off a sterile sample of the urine for laboratory analysis, and a drain is provided which is operable without contacting the urine.

The flow rates determined by the microprocessor are subjected to post-processing, wherein retrospective averages of the flow rates are taken for a certain block of predetermined intervals during which the rates were being generated, prospective averages are determined for a second block for such predetermined intervals with the second block having an overlap with the first block, and combined averages are generated by merging the retrospective and prospective averages for the overlap.

**Description**

## URINE COLLECTION MONITOR

The present invention relates to a method and an apparatus for determining the flow rate of urine during micturition and for determining other variables, and for obtaining a urine sample for clinical laboratory analysis.

Measurement of urinary flow rates (uroflowmetry) during micturition is an important technique of evaluating lower urinary tract dysfunction. Average flow rates and the peak flow rate, along with the patterns of changing flow rates during the micturition event allow physicians to identify conditions of outflow obstruction and differentiate between anatomic and neurologic disease.

The simplest form of uroflowmetry is observation of the urine stream by a trained physician. A simple and more quantifying technique is timing the voiding of a patient while collecting the urine into a calibrated container, thereby allowing average flow rate to be calculated. Other devices allow peak flow rate to be estimated by collecting urine into chambers of varying sizes in a device, with the filling of the chambers dependent upon flow rates. Observation of urination, however, is embarrassing to many patients, and many cannot void or do not void normally under such conditions. Furthermore, observation, timed voiding, and peak flow measurement alone give only partial information about how the patient urinates.

Electro-mechanical devices have been devised to make a recording of urine flow rates during micturition. Many technologies have been used to make the measurement. Measurement by weight is a common technique. Weight represents volume, and change in weight over time represents flow rate. Other technologies include micro-turbines in which the urine flowing through a tube acts upon a small fan blade, the rotation of which is proportional to urine flow rate, with the rotation being measured optically. Another technique employs a DC motor with a blade rotating at a fixed speed. The urine acts upon the blade to impede its rotation causing the motor to draw more current to rotate the blade at a fixed speed. The change in current draw is then measured and reflects flow rate. Other techniques reported include measurement of the electrolytic properties of urine, measurement of the cooling effect of the flow of urine on a heated electrode, and occlusion of a $CO_2$ valve by the urine stream. Further, in the past a capacitor has been used to measure volume in a cylinder with change in volume representing flow rate. However, such a device has not been acceptable in practice because of the errors which result in certain portions of the data generated.

With the exception of the weight transducer method, all the other techniques require the urine to come into contact with the sensor mechanism. This has several shortcomings. First, the sensors must endure repeated exposure to urine, which is corrosive and damaging to the sensors. Cleaning is of utmost importance to maintain reliable performance of the sensor. Cleaning is also important in that infected urine may remain in or on the mechanism, allowing bacteria to grow and exposing other patients to disease. Further, since urine from several patients comes into contact with the sensor mechanism, the urine cannot be used reliably for clinical chemical and microbiological analysis. On a practical basis, the sensor systems cannot be cleaned adequately to provide the level of cleanliness required for such urinalysis.

While the urine does not directly contact weight-type sensors, similar problems exist. Urine is collected into a vessel which rests on the weight sensor. The sensor must be isolated from patient contact, and the urine must be directed into the collector from a standard size and height channel such that the weight measurement is not changed by the kinetic forces of the urine stream. Therefore a weight-type apparatus must provide a means of directing the urine into the collection cup. Since urine contacts this portion of the apparatus (generally a large funnel), this system obviates the use of the voided urine for additional laboratory tests.

Patients requiring both urinalysis test and urine flow rate measurement now must void twice during the visit to the physician's office or clinic. This presents a practical problem, and in some cases patients try to hydrate quickly by drinking excessive amounts of fluid. This can lead to short-term metabolic imbalances which can distort the results of urinalysis. Many patients requiring the urine flow test also require urinalysis. Obstruction and infection are frequently concurrent and may have causal relationships. Outflow obstruction can cause high pressure in the bladder during micturition resulting in reflux of urine into the kidneys. This may result in impaired kidney function which is measured through chemical urinalysis.

Summary of the Invention

The present invention provides a method and apparatus for flow analysis during collection of a urine sample, and for simultaneously maintaining the urine sample sterile for use in clinical and microbiological analysis. A collector assembly adapted to fit a standard commode is used and includes a fold-out liner covering the entire target area for capturing the urine stream during voiding. A variable capacitor is included in the collector assembly, which has a capacitance value which varies with the amount of fluid in the collector assembly. The variable capacitor is attached to a capacitance bridge, and the change in capacitance is provided as a voltage to a microprocessor for determining flow rates and other, related variables. A tube is provided for aspirating a urine sample from the collector for urinalysis, so that the sample is not handled by a person. The collector assembly also includes a drain mechanism allowing the unwanted urine to be drained into the commode without handling the assembly itself and protecting the operator of the apparatus from contact with the urine. The collector assembly is provided as a single unit for easy disposal when a given sample has been taken.

The variable capacitor has air acting as a dielectric, so that as the collector assembly fills with fluid, the capacitance changes.

The method of the invention involves determining a potential difference between two points in the capacitance bridge, with the potential difference relating to the changing capacitance value. This potential difference is processed for input into a microprocessor, which samples the processed potential difference periodically, thus determining the flow rate. Other variables which are determined are the peak flow rate, average flow rate and total time of micturition. A printer is connected to the microprocessor for outputting the flow versus time results, and a control panel is provided for either interface.

Brief Description of the Drawings

Fig. 1 is a perspective view of a commode utilizing the invention;

Fig. 2 is an elevation, partly in section, of a portion of the commode of Fig. 1;

Fig. 3 is an enlarged view of a portion of Fig. 2;

Fig. 4 is a view taken along line 4-4 of Fig. 3;

Fig. 5 is a view similar to Fig. 3, showing an alternative embodiment of the variable capacitor of the invention;

Fig. 6 is a view taken along line 6-6 of Fig. 5;

Fig. 7 is a block diagram of the processing circuitry of the invention;

Fig. 8 is a schematic diagram of a capacitance bridge used in conjunction with the circuitry of Fig. 7;

Fig. 9 is a schematic diagram of a synchronous demodulator used in the circuitry of Fig. 7;

Fig. 10 is a diagram of the input and output to a reference generator utilized in the invention; and

Fig. 11 is a flowchart of a measuring loop utilized by the invention;

Fig. 12 is a flowchart of a post-processing loop utilized by the invention;

Fig. 13 is a graph showing the flow output of a constant-flow pump and the unprocessed data for such a pump; and

Fig. 14 is a graph showing the steps in post-processing for the data of Fig. 13.

Description of the Preferred Embodiments

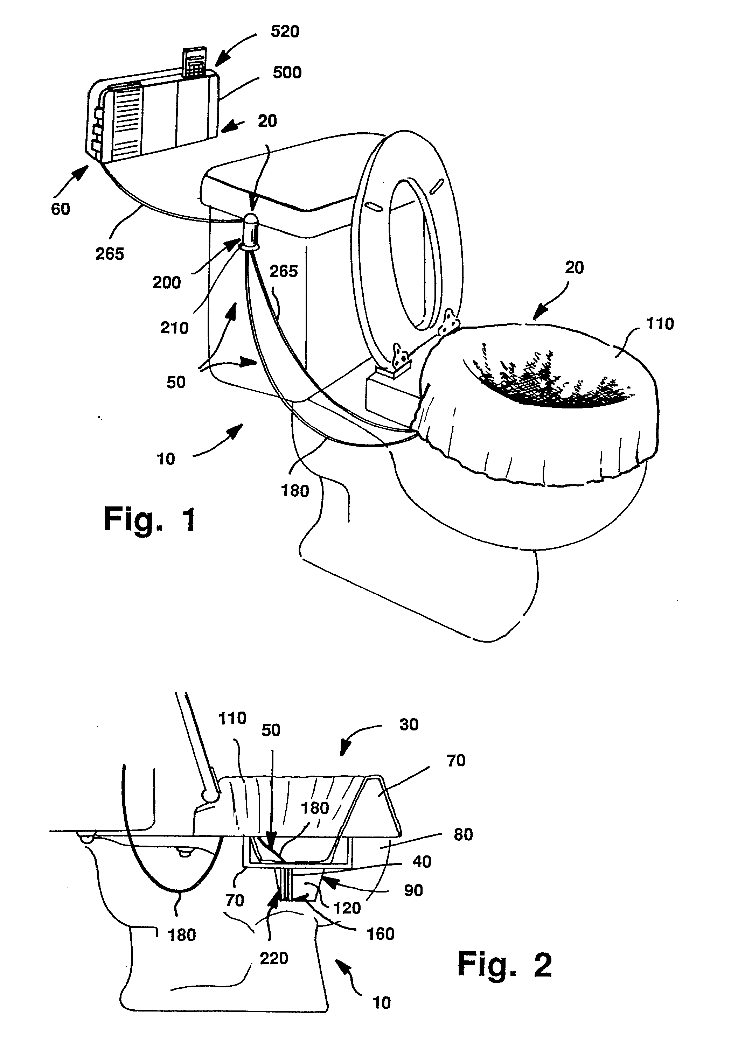

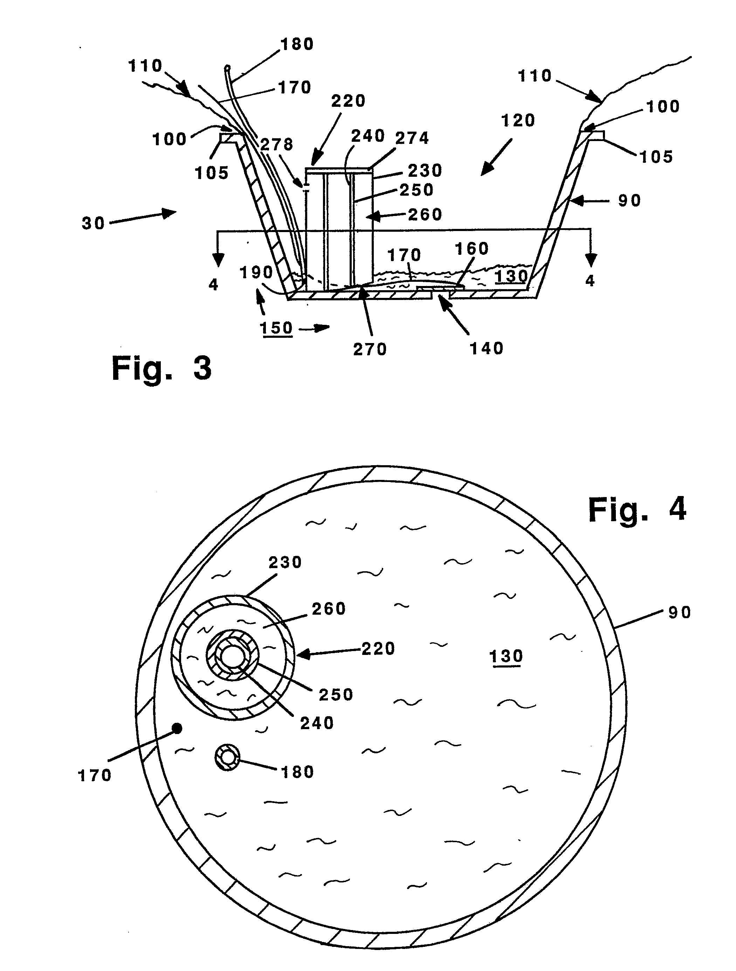

Figs. 1 and 2 show a typical toilet or commode 10 in connection with which the method and apparatus of the invention are used. The urine collection monitor 20 includes a urine collection system 30, a sensor 40, a urine sampling system 50, and an electronics module 60. The urine collection system 30 includes a commode adaptor 70 which is configured to fit over a standard toilet bowl 80. The adaptor 70 is a reusable item which may be left in place on the bowl 80. The collection system 30 also includes a vessel 90, which is basically bucket-shaped, and has a rim 100 (shown in Fig. 3) to which a liner 110 is attached in a fluid-sealing fashion. The rim 100 has a lip 105 for supporting the vessel 90 when it is inserted into the adaptor 70, which is preferably configured to the outside shape thereof.

Referring to Figs. 2 and 3, the vessel 90 has an interior 120 in which a fluid 130, such as a patient's urine, is collected during micturition. (As will be apparent in the course of the following discussion, the principles of the invention are not confined to the specific application of uroflowmetry.) A drain is provided, comprising an aperture 140 which provides fluid communication between the interior 120 and an exterior 150 of the vessel 90. A plug 160 is provided for sealing the aperture 140, and a cord 170 or other means of pulling the plug 160 is provided. The cord 170 has one end attached to the plug 160, and the other end extending to the exterior of the urine collection system 30, so that when a sufficient sample has been collected and the necessary measurements have been taken, the plug 160 may be pulled open as shown in Fig. 2 so that the urine flows out of the aperture 140 and into the commode 10, in order to dispose of the unwanted portion of the urine.

The plug 160 may comprise a piece of tape configured to seal the aperture 140, and the pulling means 170 may include a piece of tape with an end thereof attached to the plug 160, such that pulling of the pulling means 170 will shear or rupture the plug 160, allowing for draining of the fluid 130.

The use of the present device with its commode adaptor 70 allows the patient to void in a bathroom, rather than in a special apparatus set up in an examination room. This has the advantage of reducing deleterious psychological affects which may cause the patient to void in an abnormal fashion.

The urine sampling system 50 includes a conduit or tube 180 with one end having an opening 190 in communication with the interior 120 of the vessel 90, preferably near the bottom of the vessel. The other end of the tube 180 is connected to a means for drawing a sample from the interior 120 of the vessel 90, such as an evacuating device 200 of a type known in the art, which may be the VACUTAINER® evacuated test tube device produced by Beckton Dickinson and Co. of Rutherford, New Jersey, which includes a pre-evacuated receptacle 210. The tube 180 is connected by the user of the system to the receptacle 210, and has a seal adjacent thereto. The device 200 includes a needle (not separately shown) for puncturing the seal of the tube 180. Thus, when the patient has voided, the user of the monitor 20 punctures the seal, and the vacuum in the device 200 draws a sample of urine 130 from the interior 120 through the tube 180 into the receptacle 210. The receptacle 210 is then removed from the device and taken to a laboratory for urinalysis. Other means of drawing a sample may of course be utilized.

Since a urine sample may be aspirated through the conduit 180 without being handled by the physician, nurse or other operator of the device, the operator never needs to come in contact with urine, which may be infected. Similarly, the operator is protected from contacting the urine by means of the cord 170 which is utilized to drain the vessel 90 from a point remote therefrom, again preventing unwanted contact with potentially infected urine. It will also be appreciated that the vessel 90 and lining 110 shield the patient from the commode adaptor 70 and the commode 10 itself, thus protecting patients from possible contamination from earlier uses by other patients.

As shown in Figs. 3 and 4, carried in the interior 120 of the vessel 90 is a variable capacitor 220 having a first electrode 230, a second electrode 240, a first dielectric 250 disposed around said second dielectric 240, and an air space 260 between the first electrode and the first dielectric, with the air space 260 acting as a second dielectric.

The first and second electrodes 230 and 240 and the first dielectric 250 are in this embodiment disposed in the interior 120 of the vessel 90. The first electrode 230 is configured as shown in Fig. 3 so as to allow communication of the fluid 130 with the air space 260, such as by including an aperture 270. Thus, as the vessel 90 is filled, urine 130 displaces air in the air space 260, thereby altering the capacitance value of the capacitor 220.

The top of the variable capacitor 220 may be open, in order to allow air present in the air space 260 to escape as it is displaced by incoming fluid. Otherwise, there would be resistance to the incoming flow, and possible turbulence due to bubbling as the air escapes past the fluid 130 at the bottom of the variable capacitor 220. Alternatively, in the preferred embodiment a cap 274 is provided atop the variable capacitor 220 to prevent splashed fluid or other materials from entering the air space 260 except through the aperture 270. In this embodiment, a second aperture 278 is provided in the electrode 230, to allow air to escape the air space 260, which would otherwise be inhibited by the cap 274.

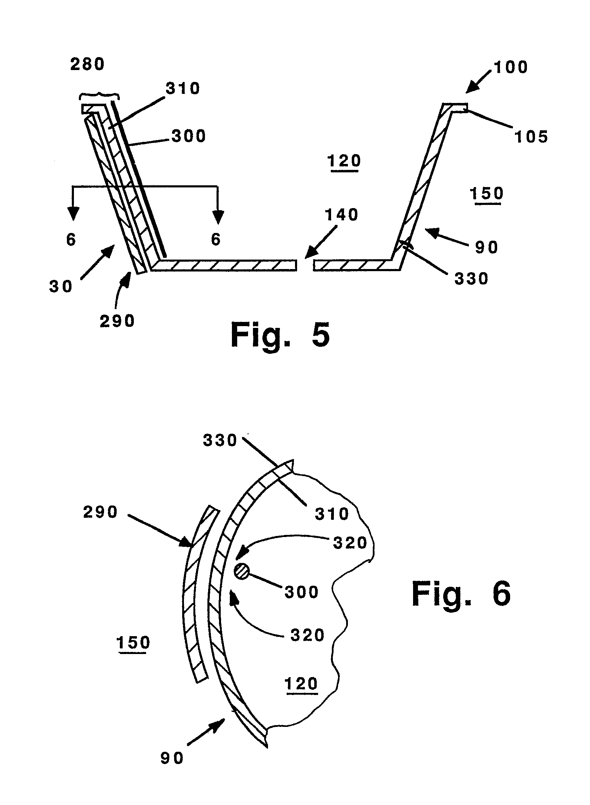

An alternative embodiment of the invention utilizes a variable capacitor 280 as shown in Figs. 5 and 6. The capacitor 280 includes a first electrode 290, a second electrode 300, a first dielectric 310, and an air space 320 comprising a second dielectric of the capacitor 280. In this embodiment, the first dielectric 310 comprises a portion of a wall 330 of the vessel 90 defining the interior 120 and the exterior 150 of the vessel. The first electrode 290 is thus disposed adjacent the first dielectric 310 at the exterior 150 of the vessel 90. Urine 130 is therefore prevented from contacting the first electrode 290. In both embodiments of the variable capacitor (220 and 280), the electrodes may be made of brass, aluminum, or other conductors.

The second electrode 300 of the capacitor 280 may simply be a length of wire or a strip of conductor in tape form adhered to the inside of the vessel 90, and may or may not be electrically insulated from the fluid 130. The capacitor 280 exhibits relative independence of resistivity of the fluid, and therefore is relatively independent of the type of fluid which acts to vary the capacitance value thereof.

In the embodiments of Figs. 3-4, the vessel 90, the variable capacitor 220, the liner 110, the plug 160, the cord 170, and the tube 180 comprise a single disposable unit. Thus, when one patient has used the collection system 30, and the appropriate data have been taken and a sample has been obtained, the plug 160 is pulled as described above, and then the tube 180 is detached from the receptacle 210, and the tube 180, the cord 170 and the liner 110 are stuffed into the interior 120 of the vessel 90 and the entire system 30 may be disposed of. Similarly, in the embodiment of Figs. 5-6, the vessel 90, the lining 110, the second electrode 300, the cord 170 and the tube 180 comprise a single unit which may be disposed of once it is used. In the embodiment of Figs. 5-6, the first electrode 290 may be either made a fixed part of the unit described above (such as a conductor in tape form or otherwise adhered to the vessel 90)--and therefore would be disposed of along with the vessel--or the first electrode 290 may alternatively be attached to the commode adaptor 70, depending therefrom for alignment with the wall 330 of the vessel 90 when it is placed in the commode adaptor 70 as in Fig. 2. The latter embodiment has the advantage of economy, since the first electrode 290 would be utilized repeatedly.

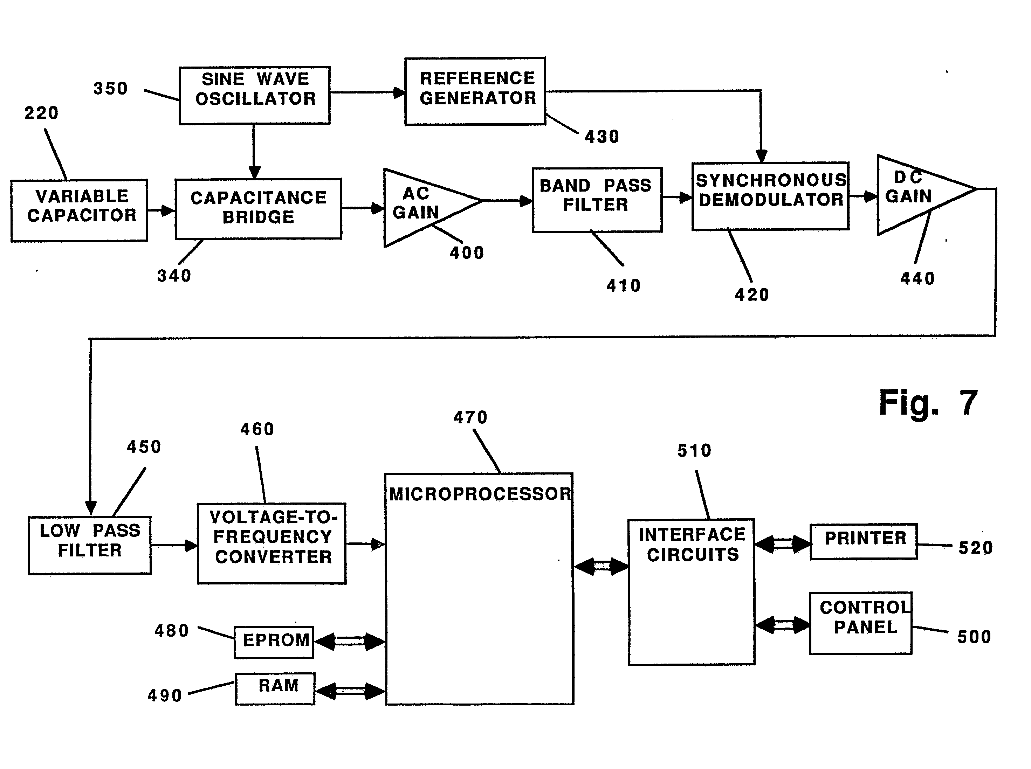

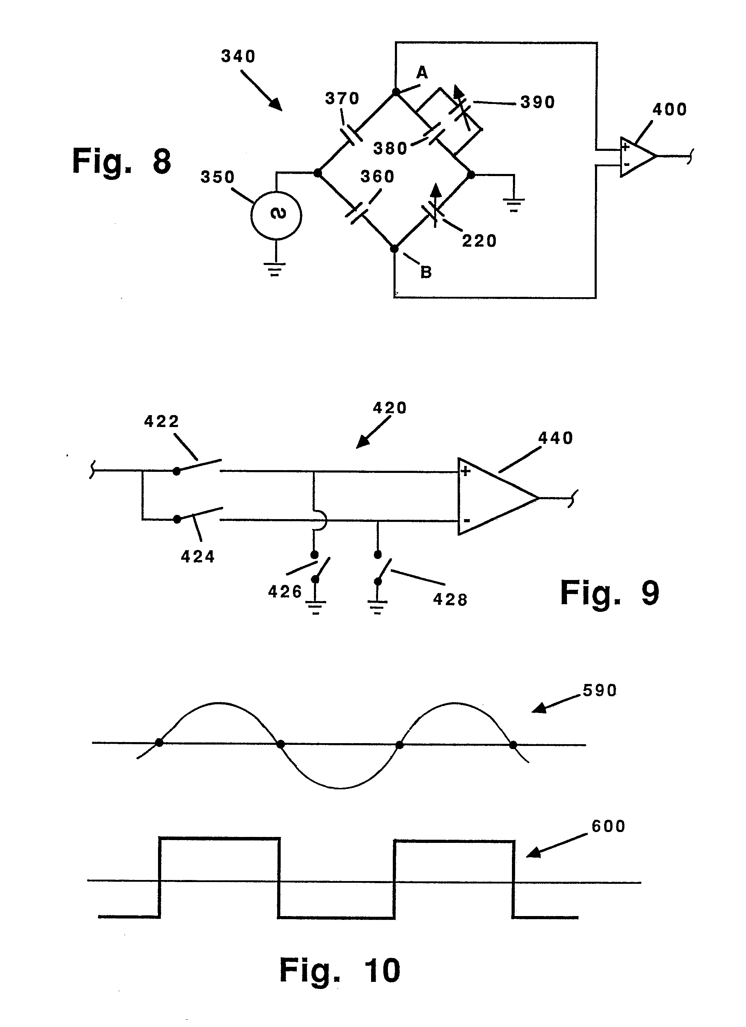

The electrodes 230 and 240 of the variable capacitor 220 are electrically connected to a capacitance bridge circuit 340, as represented in Figs. 7 and 8. This is done automatically when the user places the vessel 90 into the commode adapter 70, with both the vessel 90 and adaptor 70 being provided with contacts (not shown), so that when the vessel 90 is in place, the proper connections are made. A coaxial cable 265 (see Fig. 1) is provided for connecting the capacitor 220 to the module 60. In the course of the following discussion, any reference to the capacitor 220 may equivalently refer to the capacitor 280 or other variable capacitor configurations which would operate under the same principle.

All of the components shown in the block diagram of Fig. 7 are preferably carried by the electronics module 60, except for the variable capacitor 220. However, any desired portion of the ciruitry may alternatively be carried by the commode adaptor 70.

A sine wave is provided by an oscillator 350 to the capacitance bridge 340, which includes, in addition to the variable capacitor 220, fixed capacitors 360, 370 and 380. When the capacitance values of the capacitors 220, 360, 370 and 380 are all equal, the potential difference between a first point A and a second point B of the circuit is zero. A differential operational amplifier 400 is connected to points A and B. Thus, when the capacitance values of the capacitors 220, 360, 370 and 380 are equal, no potential difference appears at the amplifier 400, which therefore has a zero output. (In the preferred embodiment, for practical reasons discussed below a small imbalance to the capacitance bridge 340 is deliberately provided by a trim capacitor 390, so that at least a small positive voltage always appears at the input to the amplifier 400.)

When fluid begins to fill the vessel 90, the capacitance of the variable capacitor 220 changes, thereby unbalancing the capacitance bridge 340, causing an increased potential difference between points A and B to appear at the amplifier 400. This potential is directly related to the amount of fluid in the vessel 90--and indeed is substantially proportional thereto--and thereby provides a means for measuring the same.

The output of the amplifier 400 is input to a band pass filter 410, which in the preferred embodiment passes frequencies frequencies in the range of about 500 Hz to 50 kHz, and minimizes any phase shift at the center frequency of approximately 5 kHz. This filter 410 operates to filter out line noise, radio frequency interference, and other high frequency noise, which may emanate from sources external to the monitor 20, or may emanate from other components of the monitor itself.

The signal, after filtering in the band pass filter 410, is input to a synchronous demodulator 420, which also has as an input an output from a reference generator 430 which is in turn driven by the sine wave oscillator 350. The synchronous demodulator 420 effectively rectifies the signal input thereto, obtaining a DC equivalent of the AC sine wave from the filter 410. As the vessel 90 fills, this signal grows in amplitude. The demodulator converts the input signal without significant phase shift, and with fast transitions.

This demodulator 420 typically requires a square wave input from the reference generator 430. For this purpose, the reference generator 430 amplifies the signal from the oscillator 350, forming very steep transitions. Thus, if the signal is centered on zero volts, these steep transitions will occur around the zero crossings of the wave. An example of the intended conversion is shown in Fig. 10, with the waveform 590 representing an idealized input to the reference generator 430, and the waveform 600 representing the idealized output therefrom. In the preferred embodiment of this invention, the signal is actually centered on a positive voltage, with the minimum voltage being approximately zero; the demodulator functions equivalently for such a signal as for a signal centered on zero volts.

The reference generator 430 may use a Schmitt trigger, which actually detects points on the signal obtained from the oscillator 350 which are adjacent, but no quite at, the zero crossings. The output of the generator 230 is an approximately 5 kHz wave in phase with the sine wave input thereto. The generator 430 could also be used to generate a signal 180° out of phase with its input for use by the synchronous demodulator, if desired, so along as the zero crossings of the signal match up.

A schematic diagram of the synchronous demodulator 420 is shown in Fig. 9. As shown in Figs. 7 and 9, the output of the synchronous demodulator 420 is provided as input to a DC amplifier 440. The demodulator includes four switches 422, 424, 426 and 428, which are controlled in a conventional manner to open and close in response to the sign of the input to the demodulator. In the preferred embodiments, the switches 422, 424, 426 and 428 are CMOS devices, although they could alternatively be bipolar devices. When positive input--i.e. a positive portion or lobe of the AC wave input--is received by the demodulator 420, switch 422 is on (i.e. closed) to feed the positive signal into the positive side of the amplifier 440. Switch 424 is off (i.e. open) to prevent the signal from reaching the negative side of the amplifier 440. Switch 426 is off, again to allow the positive signal to reach the amplifier 440, and switch 428 is on, in order to prevent any other signal, such as unwanted spurious signals, from reaching the negative side of the amplifier 440.

Conversely, when a negative input in received by the demodulator 420, switch 422 is off, switches 424 and 426 are on, and switch 428 is off. This feeds the negative signal to the amplifier 440, while preventing any signal from reaching the positive side of the amplifier. The result of this configuration is that the demodulator 420 has the effect of rectifying the input thereto. Rectification can, of course, also be accomplished with a diode, but the output will not be synchronous, that is, it will not maintain the phase characteristics as accomplished by the demodulator 420.

The demodulator 420 thus eliminates components of the input signal which are out of phase, and external noise not synchronous with the oscillator output is eliminated. The result is that the output of the synchronous demodulator 420 is the DC equivalent of the sine wave output from the capacitance bridge 340, wherein the amplitude is proportional to the amplitude of the AC signal, and therefore proportional to the change in capacitance of the variable capacitor 220.

The amplification by the amplifier 440 is preferably relatively small in order to prevent amplification of parasitic DC parameters, such as DC bias inherent in the devices used. The gain of the amplifier 400 may on the other hand be relatively large (and in the preferred embodiment gain of about 30 is used), since DC offset bias is effectively rejected in an AC carrier system.

The output of the amplifier 440 is then preferably passed through a low pass filter 450, in order to in effect average out the wave, giving a true DC equivalent thereof, and further excluding unwanted noise from the signal.

The signal is then input to a voltage-to-frequency converter 460 which is a simple, low cost way to feed the signal into the microprocessor 470. Alternatively, an analog-to-digital converter could be used for feeding digital data in a serial or parallel bit stream, with the digital numbers being proportional to the DC signal input. The microprocessor 240 is preferably a processor such as the INTEL 80C39. A single-line bit port is utilized for feeding in the output from the converter 460.

As mentioned above, the capacitance bridge 340 is given a slightly positive DC offset voltage when the vessel 90 is empty. This is done in order to accommodate any lack of tolerance of the voltage-to-frequency converter 460 to negative voltages. If the potential difference between points A and B in Fig. 8 is exactly zero when the vessel 90 is empty, it is possible that DC bias somewhere in the system could actually make the voltage output from the amplifier 440 become negative, when it should be zero. Thus, a slight offset bias of approximately 170 mV in the preferred embodiment is utilized. Because of the offset bias induced by the trim capacitor 390, the amplifier 440 has a small output even when the vessel 90 is empty.

The microprocessor 470 samples the output of the voltage-to-frequency converter 460 at regular intervals. The output of the converter 460 is essentially a square wave signal with a frequency proportional to the voltage which was input to the converter 460. The input to the converter 460 should, as noted above, being non-negative, and therefore the output of the converter 460 is preferably a signal with amplitude between zero and 6 volts, where approximately 3 volts represents a "zero crossing" or frequency transition.

The microprocessor 470 includes an internal counter governed by an internal clock, with the counter generating bits related to the frequency readings at the input. The clock has a count rate which in the preferred embodiment is five counts per second, so that each count is taken one-fifth of a second after the preceding count. Typically, the frequency of the input may be 5 to 40 kHz, and thus at a counting rate of five times per second, counts of 1,000 to 8,000 may be expected. For this reason, the internal counter of the microprocessor 470 preferably has 16 bits available for each one-fifth second count, with a minimum of 13 bits being necessary to accommodate 8000 binary-encoded counts (since $2^{13} = 8192$).

The functions of the microprocessor 470 are controlled by a program stored in an EPROM (erasable programmable read-only memory) 480. As each one-fifth second reading is made by the microprocessor 470, the difference between the current count (which is related to the volume of fluid in the vessel, and therefore may be referred to as a volume reading) and the previous count is calculated. This differential count represents the change in volume between the times of the two intervals, and thus represents the flow rate for that period of time. The differential count may be referred to as a flow count or flow reading.

In order to effect the comparison of current and previous volume readings, an internal register (not separately shown) is provided in the microprocessor 470 for storing the previous volume reading at any given time, and the current volume reading for a given interval is substituted therefor as the next succeeding count is made.

The differential counts are stored in the RAM (random-access memory) 490. This repeated for a predetermined length of time, preferably ninety seconds, since a patient will typically void in less than this time. Thus, a total of 450 counts, at five counts per second for ninety seconds, are taken of the frequency input to the microprocessor 470.

Included as Appendix A hereto is a copy of software code for the microprocessor 470 which may be utilized to implement the present invention.

A control panel 500 is provided for user interface with the present system, with interface circuits 510 for interfacing between the control panel 500 and the microprocessor 470. The control panel 500 preferably includes an ON switch, a RESET switch and a PRINT switch. In addition, lights are provided, including an ON light, a READY light, a COMPLETE light, and a LOW BATTERY light.

When the user of the system and the patient are ready to begin, the user presses the ON switch, at which time the microprocessor 470 goes through an initialization procedure, clearing the RAM 490, the various ports of the processor 470, and the output of the microprocessor 470. Other standard initialization procedures may be implemented. The internal counter of the microprocessor 470 is also initialized and, as noted above, is programmed for making counts every fifth of a second, by way of clock interrupts.

After initialization, the the power for the analog circuitry (i.e., the circuitry including the devices shown in Fig. 7 between the capacitor 220 and the converter 460, inclusive) for the system is turned on by the microprocessor 470. Preferably the system is driven by battery power for independence from AC outlets; and thus, postponing the turning on of the analog power saves battery life. The battery is then automatically checked, and if the voltage is low, the LOW BATTERY light is turned on, and the procedure is halted. The microprocessor 470 may be programmed to wait a certain period of time for the analog circuitry to stabilize (ten seconds usually being sufficient), and implement another battery check, again turning on the LOW BATTERY light if the voltage is low.

If the battery voltage is sufficient, the microprocessor 470 then checks for whether a vessel 90 is in place. If no vessel 90 is in place in the adaptor 70, then the capacitor 220 will be missing from the bridge circuit 340, and the circuit 340 will be seriously out of balance. This will result in a frequency input to the microprocessor 470 considerably out of the expected range, which may, for instance, be 2 to 9 kHz. If no vessel 90 is detected, the READY light is flashed or blinked on and off repeatedly to indicate this.

Meanwhile, the battery is checked again, since it is now under load, and again, if the voltage is low, the LOW BATTERY light flashes on and off, and the procedure is interrupted.

If the vessel 90 is in place, and the battery is found to be in good order, then the microprocessor 470 stores (in the RAM 490) the initial count rate as the count relating to the initial volume of zero in the vessel 90. (Even with the vessel empty, this initial count will be nonzero, because of the offset bias in the capacitance bridge 340.) This automatically accounts for any differences in tolerances of the variable capacitor 220, which may result in slightly different "zero" frequency counts to the microprocessor 470 for each disposable collection system 30. In effect, storing the initial count rate as the empty-vessel reading calibrates the system for the upcoming measurements of flow rates and total flow volume. Then the READY light is turned on (but not flashed, as when there was no vessel 90 in place).

The microprocessor 470 then initializes the RAM 490 to its initial address, and a loop is implemented by the program in the EPROM 490 to detect when the patient begins voiding. In order to do this, the microprocessor 470 determines when a change in the frequency count obtained from the converter 460 occurs. Preferably, a minimum difference in counts between successive fifth-second counts at the microprocessor 470 input is awaited, such as a minimum difference of 4 counts, which in the preferred embodiment relates to a flow of 2 milliliters per second. The relation between the number of counts and the actual volume of fluid in the vessel may, of course, be calibrated differently if desired.

Once again, while the minimum difference is awaited, the battery is checked as above.

Once the minimum difference is detected, the READY light is turned off, and an optional MEASURING light on the control panel 500 may be turned on. At this point, the microprocessor 470 begins storing the one-fifth second differential counts (i.e. the flow readings) into the RAM 490. The above-mentioned internal register is used by the microprocessor 470, so that when the minimum difference mentioned above is detected, the count previous to that which resulted in the minimum difference may be stored in the RAM 490, for later use in determining the total flow volume.

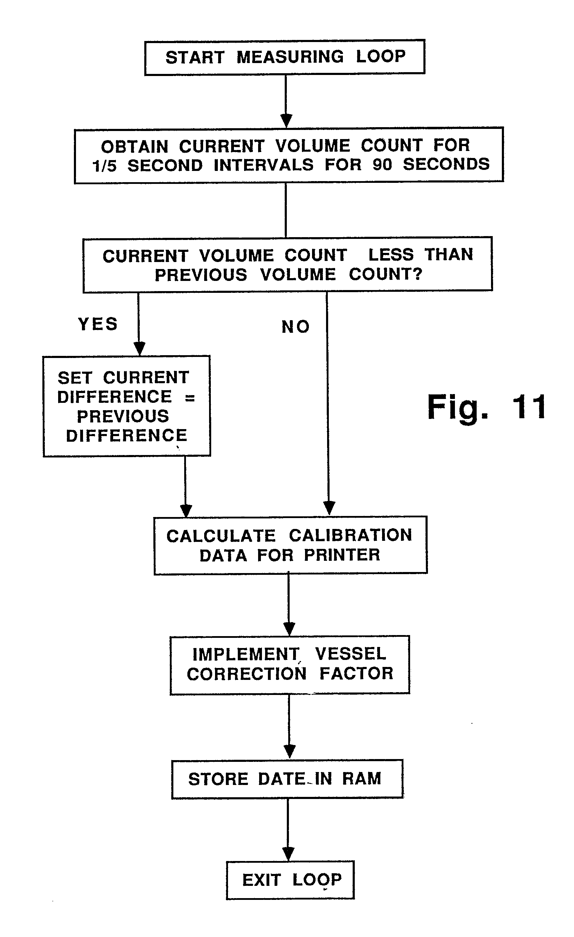

The program from the EPROM 480 then implements a loop for measuring and storing the incoming flow readings into the RAM 490. This loop is represented in the flow chart of Fig. 11. In this portion of the program, the current counts for a one-fifth second interval are obtained from the converter 460 by the microprocessor 470. As noted above, the microprocessor begins to store the flow data as soon as a minimum difference between two successive counts is detected.

The program will also compare successive volume readings to determine whether a given volume reading for a fifth-second interval is less than that of the interval before. Since the capacitance of the capacitor 220--and hence the DC voltage output from the amplifier 440 and the frequency output from the converter 460--is proportional to the total amount of fluid in the vessel 90, as the vessel fills up the frequency output from the converter 460 should rise continuously. However, there will necessarily be motion of the fluid 130 in the vessel 90, and therefore wave action or other disturbances may cause the amount of fluid 130 in the vicinity of the capacitor 220 to be less at a later time (when the vessel 90 actually has more fluid in it) than at an earlier time. Such wave action would therefore introduce errors into the data; and since it is certain that the amount of fluid 130 in the vessel 90 at a later time is always at least equal to the amount of fluid present at an earlier time, the program is designed to correct for this type of error. Therefore, if the volume reading for a given interval is less than the volume reading for the preceding interval, then the current differential reading is set to equal to the previous differential reading. The effect of this is to create a presumption that the flow remained constant for that interval (since it could not have been negative, and was presumably not zero). Although this may not precisely reflect the actual flow of fluid into the vessel 90, as a result of the error introduced by wave action, as will be seen in the discussion below regarding post-processing of the data, any error introduced into the flow data is minimized by the post-processing of the data. Also, the total volume reading which is finally obtained is not affected at all, since the calculation for the total volume reading is based solely upon the initial and final volume counts.

The differential counts are then converted to the chosen calibration of a display device such as printer 520. For instance, if the printer is a dot matrix printer, the data may be calibrated to store a certain number of dots per frequency count obtained from the converter 460.

Preferably, the program includes a routine to correct for various shapes of vessels 90. For instance, since the sides of the vessel 90 as seen in Figs. 2 and 3 are sloped outwardly (relative to the bottom of the vessel), a frequency count increase (which relates to an increase in volume of the fluid 130 in the vessel 90) will be less at a later time than at an earlier time for a given increase in volume of the fluid, since the height of the fluid will rise less where the walls are farther apart, namely at the upper end of the vessel 90. Therefore, the program corrects for this in the following manner. As noted above, the frequency from the converter 460 is proportional to the total amount of fluid 130 in the vessel 90. Thus, where the frequency is low, a difference between two successive interval counts will indicate a lower total volume change than the same count difference when the total volume is greater. Therefore, the program is designed to determine the total frequency count, and for a given frequency count, to then determine by means of correction factors what total volume change the count difference between two successive intervals represents. This is done the entire time the data is being processed during the ninety-second test.

The correction factors for the vessel 90 may be determined in either empirically or mathematically. For instance, for the generally conically-shaped vessel such as vessel 90, it is straightforward and well-known to calculate the changes in volume as the vessel fills with a fluid as a function of the height of the fluid within the vessel. For more complicatedly shaped vessels, an empirical determination of this function may be preferable. The correction factors are stored as part of the program, either in the form of conversion tables or as a conversion formula.

Once the flow counts have been calibrated and corrected, they are stored in the RAM 490, and the process is repeated for the ninety-second test period or until the test is interrupted by the user (such as by pressing the PRINT switch). The loop is then exited, the MEASURING light is turned off and the COMPLETE light is turned on. At this point, the volume count for the last one-fifth second interval for which a measurement was taken is stored as representing the final volume of the fluid 130 in the vessel 90. The internal timer for the microprocessor 470 and the counter are then automatically switched off, as is the power to the analog circuitry.

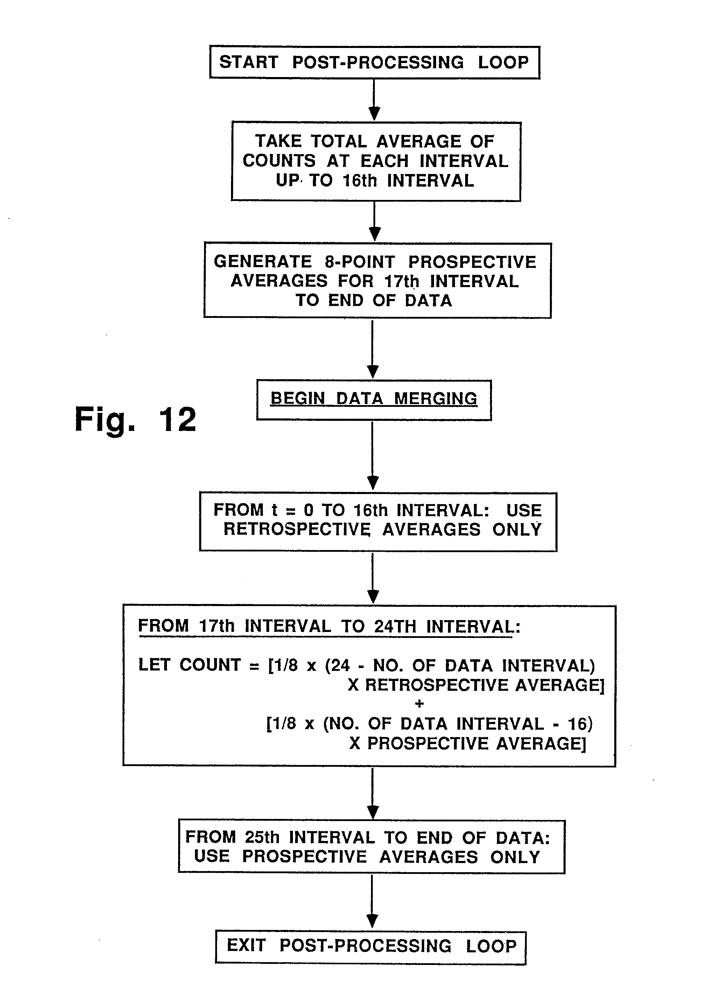

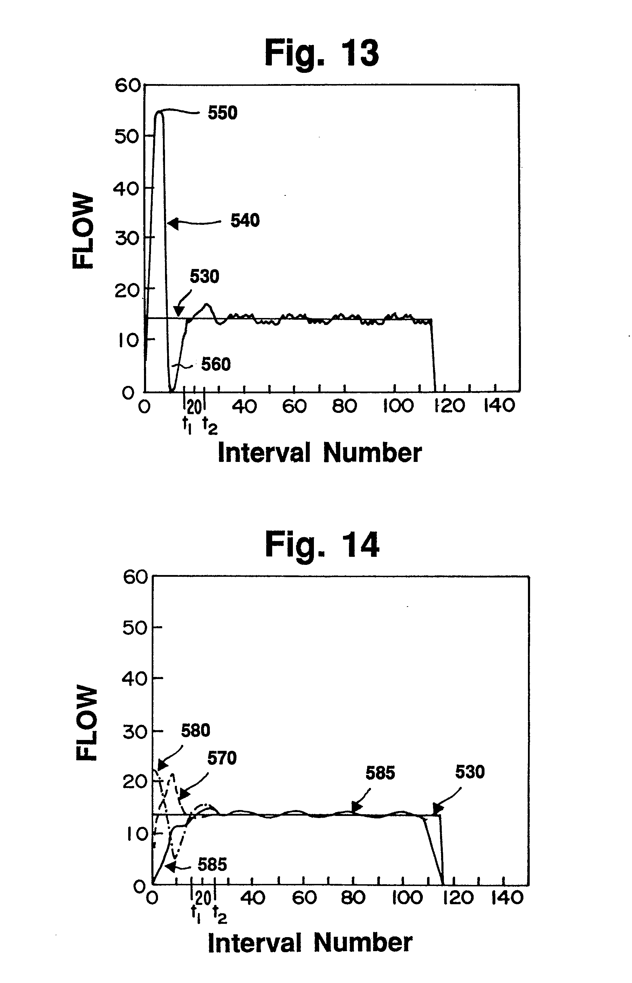

At this point, in the RAM 490 are stored the flow counts for the entire test, corrected and calibrated as noted above, as well as the initial and final volume counts, each of the stored counts is correlated with the interval to which it pertains. A post-processing loop is then implemented, as represented in the flow chart of Fig. 12. The need for the post-processing loop is explained by reference to the graph shown in Fig. 13, which is based upon actual data. As mentioned above, the flow rate of fluid 130 into the vessel 90 is represented by the successive differences between counts obtained from the converter 460. Curve 530 shows the flow output from a test pump which is designed to produce a constant flow. Thus, flow begins at time zero, increases to a certain constant, and then diminishes after a given period of time. However, when fluid (whether from a pump or from a patient) reaches a vessel 90, it changes the effective geometric configuration of the variable capacitor 220, because of the fluid's conductive properties. Thus, the capacitance value of the variable capacitor 220 changes very quickly at first, causing a spike in the data. A representative graph of the actual, unprocessed data from a constant flow pump appears as curve 540 in Fig. 13, with the peak of the spike being designated as 550.

The spike 550 in the unprocessed flow data is typically followed by a trough 560, which is a result of the artificially high spike 550; since the spike 550 caused inaccurately high frequency counts, the actual frequency counts later obtained will seem to indicate a large drop in the flow rate, when in fact, in the example given, the flow rate is constant. The "hook" in the curve 540 caused by the spike 550 and trough 560 is a problem resulting from the use of variable capacitors such as capacitor 220 in conjunction with a dielectric such as a fluid which actually may conduct current. This type of erroneous output has heretofore made the data obtained at the beginning of the flow period unusable, and because of this, the use of a variable capacitor in a fluid monitoring system has heretofore been impractical and unreliable. The problem is now solved by the present method of post-processing the flow data, as described in detail below.

The actual flow output of a patient during voiding will not be constant, as represented by curve 530, but more typically resembles a bell curve. However, the same type of "hook" error is introduced no matter what the actual flow of the pattern is; and the data errors discussed above are resolved by the present method for any flow pattern.

The post-processing method corrects for errors in the flow readings in three basic steps. First, retrospective averages for the flow readings of a first predetermined block of the intervals are generated, and prospective averages for the flow readings for a second predetermined block of the intervals are also generated, where the second block has an overlap with the first block. Then, combined averages are generated for the intervals relating to the overlap, the combined averages being derived from the retrospective averages and the prospective averages. The post-processing results in the generation of error-corrected averages, with one such error-corrected average relating to each of the predetermined intervals, which are then substituted for the flow readings in the memory.

The post-processing is carried out as follows, amd is best understood by reference to the curves shown in Fig. 14, which are based upon actual data. Each of the retrospective flow averages is generated by first generating a retrospective total of the flow readings from the first interval through the Ith predetermined interval--where I is a variable integer between 2 and a first predetermined constant J--and then dividing the retrospective total by I, and repeating the step of generating such retrospective averages for each I between 2 and J. In the preferred embodiment, J equals 24, and thus 23 retrospective averages are generated, one for each of intervals 2 through 24. (The first interval flow reading is unaltered.)

The time up through which the retrospective averages are taken is represented in Fig. 13 by $t_2$, and for this example represents 4.8 seconds worth of data (i.e., twenty-four count intervals). Thus, in order to take the retrospective averages between time zero and the 24th interval, the stored flow counts are totalled, and this total is divided by the interval number for the current count. For the seventh interval, the first seven counts are added, and this total is divided by seven. At the sixteenth interval, an average of the first sixteen data counts will be calculated, and so on through the 24th interval.

The prospective averages for the intervals between (J-K) and (N-K) are then generated, where N is the total number of intervals (450 in the preferred embodiment) and K is a second predetermined constant, equalling 7 in the preferred embodiment. Each of the prospective averages is generated by first generating a prospective total of the flow readings from the Lth interval through the (L+K)th interval, where L is a variable integer between (J-K) and (N-K). Thus, in this example, (J-K) is 17, (N-K) is 443, and L goes from 17 to 443. The first prospective average is therefore the average of the flow readings for the eight intervals intervals 17 (represented in Fig. 13 as $t_1$) through 24 (or $t_2$), and prospective averages are generated for each of the succeeding intervals through the end of the data, in each case taking the average of a given interval and the seven succeeding intervals. Of course, for the 444th through the 450th intervals, their are fewer than seven succeeding intervals, and the flow readings from these intervals are utilized only to the extent that they affect the prospective averages of intervals 436 through 443. In practice, a patient virtually never requires the full 90 seconds to void, so that there are in any case no flow readings relating to these intervals.

As the retrospective averages for intervals 1 through 16 (which equals J-(K+1)) are generated, they are stored in the RAM 490 in place of the flow readings appearing therein for those intervals. As the prospective averages for intervals 25 through 443 (i.e., J+1 through N-K) are generated, they are likewise stored in memory in place of the flow readings for those intervals. For the intervals 17 through 24 (i.e., J-K through J), the retrospective and prospective averages are first merged, according to the method represented in Fig. 12, and the combined averages are then substituted for the flow readings in memory relating to those intervals.

The method of the merging of data between the 17th and 24th data intervals as shown in Fig. 12 is as follows. Each of the combined averages is generated by adding a fraction (J-M)/(K+1) of the retrospective average to a fraction $(M-(J-(K+1)))/(K+1))$ of the prospective average, where J and K are the predetermined constants mentioned above (24 and 7, respectively), and M is the number of the interval (which may be expressed as going from (J-K) to J, i.e. 17 to 24). Thus: (J-M) goes from 7 to 0 as M goes from 17 to 24; $M-(J-(K+1))$ goes from 1 to 8 as M goes from 17 to 24 (and as (J-M) goes from 1 to 7); and the denominator (K+1) equals 8. This may be expressed as:

$$CA = ((J-M)/(K+1))RA + (M-(J-(K+1)))/(K+1))PA$$

where CA is the combined average, RA is the retrospective average, and PA is the prospective average. With the values discussed herein, the following combined averages are generated:

| Interval Number | Combined Average |
|---|---|
| 17 | 7/8(RA) + 1/8(PA) |
| 18 | 6/8(RA) + 2/8(PA) |
| 19 | 5/8(RA) + 3/8(PA) |
| 20 | 4/8(RA) + 4/8(PA) |
| 21 | 3/8(RA) + 5/8(PA) |
| 22 | 2/8(RA) + 6/8(PA) |
| 23 | 1/8(RA) + 7/8(PA) |
| 24 | 0/8(RA) + 8/8(PA) |

As the combined averages are generated, they are substituted for the flow readings in the RAM 490.

Of course, any of the variables and constants discussed above may be altered as desired, but it has been determined experimentally that the above values generate quite accurate results. Also, other details of merging the retrospective and prospective averages may be implemented without affecting the qualitative result.

The results of the data merging steps for the curve 530 are shown in Fig. 14. A graph of the retrospective averages from time zero to $t_2$ is approximately shown as curve 570, while a graph of the eight-point prospective averages is approximately shown as curve 580. It will be noted that the retrospective averages curve of 570 lags the actual curve 540 from the data received, while the prospective averages curve 580 leads the curve 540, since the prospective averages in effect anticipate upcoming data. The retrospective, prospective and combined averages generated by the merging of the retrospective and prospective averages are shown as error-corrected curve 585 of Fig. 14, which demonstrates that the method described above results in the elimination of the spike 550 and trough 560, and produces a result much more closely matching the actual output curve 530. (Incidentally, although the prospective average curve 580 is shown from time zero for illustration purposes, this prospective average is not actually utilized in the data merging until time $t_1$.)

Thus, in practice it has been found that utilization of the post-processing of the data by the method shown in Fig. 12 results in experimental curves very closely matching known flow patterns, such as the flow data represented by curve 530 for a constant-flow pump. This post-processing of data has also been performed on data relating to a bell-shaped curve such as a curve actually resulting from micturition, with results similar in accuracy to the constant-flow curve. Indeed, in the case of a bell-type curve resulting from actual urine flow data the match is even closer, for the following reason: as discussed above, the spike 550 and trough 560 are a result of the sudden effective alteration in the geometric configuration of the electrode 220 upon being contacted by fluid. If the actual flow at the beginning of data gathering is relatively low, the spike will be concomitantly low; and the converse is true. Therefore, since a constant-flow pump by definition has an initial output which is significantly large relative to its average output (indeed, it is equal thereto), the spike 550 is also large. However, human urination is such that the initial flow is small, and a maximum is reached, and the flow tapers off again--hence, the bell-shaped curve. Thus, the effect of the spike 550 and the trough 560 on the error-corrected curve 585, being at the beginning of the data, is less significant than for the constant-flow data represented in Fig. 14.

Once the post-processing loop is completed, the microprocessor 470 makes certain calculations for outputting data of importance to the physician. For instance, the peak flow rate is calculated, by storing the highest flow reading detected. Preferably, an average of the five flow readings around this highest flow reading (including the highest flow rate itself) is taken as the peak flow rate.

The time to peak flow is also calculated. The number of count intervals to peak flow is simply divided by five to determine number of seconds to peak flow (since there are five counted intervals per second).

The total voiding time is also preferably calculated, by dividing the time during which micturition was taking place by five, to obtain voiding time in seconds. It should be noted that there may be intervals between time zero and the end of the test during which no micturition is taking place. The program accounts for this in determining the voiding time by ignoring intervals during which no change in the total volume of the fluid 130 (and hence change in the capacitance value of the variable capacitor 220) was taking place. This accommodates stops and starts during micturition by the patient.

The total volume of urine 130 received by the vessel 90 is also calculated, by subtracting the initial (calibration) volume count--which relates to zero volume of fluid 130 in the vessel 90--from the final volume count. Finally, the average flow during voiding is calculated by dividing the total volume by the total voiding time as described above. The total volume calculation will, of course, depend upon the vessel correction factor discussed above, the step for which appears in Fig. 11.

Each of the parameters calculated as described above is then converted to a number relating to the desired dimensions for printout purposes. For instance, the total volume may expressed in milliliters, and for this purpose an appropriate conversion factor converting the counts to a numeral relating to actual milliliters of fluid 130 in the vessel 90 is utilized. Similarly, the average flow may be in milliliters per second, and so on. Alternatively, each of the conversions of the data may be carried out before storing the data in the RAM 490; for instance, the error-corrected flow averages generated by the post-processing method may be calibrated for the printer either before or after storage in memory. An advantage of doing so beforehand is that the time it takes to print out the data is substantially decreased, since no extra time is needed by the microprocessor 470 for conversions.

Finally, when the user presses the PRINT switch, the flow versus time graph is printed by the printer 520, and the parameters calculated above are printed out as well. A sample of the urine 130 is then obtained as described above by the evacuating device 200, the drain 160 is opened to dispose of the unwanted portion, and the physician or assistant disposes of the used urine collection system 30.

## Claims

1. An apparatus for measuring flow rates of a fluid, comprising:
a vessel having an interior for collecting an amount of the fluid;
a first electrode disposed at said vessel;
a second electrode carried in said interior of said vessel;
a first dielectric disposed adjacent said first electrode for insulating said first electrode from said second electrode and from said fluid;
a second dielectric disposed adjacent said second electrode and in communication with said vessel interior, such that said first and second electrodes and said first and second dielectrics together comprise a variable capacitor with a capacitance value which varies with said amount of fluid;
means connected to said variable capacitor for determining the flow rates of the fluid as a function of said capacitance value and for providing an output reflecting said flow rates;
means for displaying said flow rates;
means in communication with said interior for removing a sample of the fluid from said vessel; and
means in communication with said interior for draining the fluid from said vessel.

2. The apparatus of claim 1, wherein:
said vessel includes a wall defining said interior and an exterior of said vessel;
said first dielectric comprises at least a portion of said wall;
said second dielectric comprises an air space;
said first electrode comprises a first electrical conductor adjacent said wall at said exterior;
said second electrode is carried within said interior and adjacent said air space, such that as the fluid is collected in said vessel, the fluid displaces air in said air space, thereby varying the capacitance of said capacitor.

3. The apparatus of claim 2, wherein said first electrode comprises a plate conforming to an exterior shape of said vessel, and said second electrode comprises a wire vertically disposed in the interior of said vessel and near said wall substantially opposite said plate.

4. The apparatus of claim 3, wherein said first electrode and second electrode, respectively, comprises strips of conductor tape.

5. The apparatus of claim 2, wherein said removing means includes:
a tube extending into the said interior of said vessel, said tube including a closure;
means for opening said tube; and
means for drawing a sample of the fluid through said tube for transfer to a container separate from the vessel.

6. The apparatus of claim 4, wherein said draining means includes:
an aperture for providing fluid communication between said interior and said exterior of said vessel;
a plug disposed adjacent said aperture for preventing the fluid from passing through said aperture; and
means for removing the plug from a point remote from said interior of said vessel.

7. The apparatus of claim 6, wherein said removing means includes an elongate flexible means for pulling said plug, said pulling means having one end attached to said plug and having another end extending to said exterior of said vessel.

8. The apparatus of claim 3, wherein said vessel, said first dielectric, said removing means, said draining means, and said second electrode comprise a single unit for disposal of said unit when the flow rates of the fluid have been measured.

9. The apparatus of claim 2, wherein said flow rate determining means includes:
a capacitance circuit connected to said variable capacitor and including a first point and a second point, wherein said first point and said second point have a predetermined potential difference when said vessel contains no fluid and, as the fluid is collected in said vessel, have varying potential differences which vary as a function of said capacitance value;
means connected to said first and second points for detecting said predetermined potential difference and said varying potential differences between said first and second points at predetermined time intervals;
means connected to said detecting means for converting said predetermined potential difference and said varying potential differences into numerical values related to said flow rates.

10. The apparatus of claim 9, wherein:
said converting means is also for converting said predetermined potential difference and said varying potential differences into numerical values representing initial and final volume readings of said vessel; and
said flow rate determining means further includes a microprocessor for generating retrospective averages of said flow rates for a first block of said predetermined time intervals, for generating prospective averages of said flow rates for a second block of said predetermined time interval, where said second block has an overlap with said first block, and for generating combined averages for said overlap, said combined averages being derived from said retrospective averages and said prospective averages, with one said combined average relating to each of said predetermined intervals in said overlap, and for substituting said retrospective averages, said prospective averages, and said combined averages for said flow readings in said memory.

11. The apparatus of claim 9, wherein said predetermined potential difference is nonzero.

12. The apparatus of claim 1, wherein:
said first electrode is carried within said interior;
said second dielectric comprises an air space;
said second electrode is carried within said interior adjacent said air space, such that as the fluid is collected in said vessel, the fluid displaces air in said air space, thereby varying the capacitance of said capacitor.

13. The apparatus of claim 12, wherein:
said first electrode is elongate in shape and is disposed substantially vertically within said interior;
said first dielectric extends at least partially around said first electrode;
said air space extends at least partially around said first electrode; and
said second electrode is substantially cylindrical in shape and extends at least partially around said air space.

14. The apparatus of claim 13, wherein said vessel, said first dielectric, said removing means, said draining means, said first electrode and said second electrode comprises a single unit for disposal of said unit when the flow rates of the fluid have been measured.

15. A method for determining flow rates of a fluid into a vessel having an interior for collecting a fluid, wherein a variable capacitor in communication with the interior has varying capacitance values with the capacitance value at a given time being a function of the amount of fluid in the vessel at that time, including the steps of:
sampling the capacitance values at predetermined intervals for obtaining volume readings having values reflecting the amount of fluid in the interior of the vessel at each such interval;
comparing readings obtained at succeeding predetermined intervals for determining when the volume of fluid in the interior begins to increase and for calculating flow readings from the differences between successive volume readings;
for each given interval, beginning with the second predetermined interval, comparing the volume reading for the given interval with the volume reading for the preceding interval to determine whether the given interval has a volume reading which is less than that of the preceding interval, and if so, then setting the flow reading for the given interval at the value of the flow reading for the preceding interval;
calibrating the flow readings for a display;
ceasing the sampling of the capacitance values;
storing the flow readings in a memory; and
displaying the flow readings on the display.

16. The method of claim 15, further including the steps of:
generating retrospective averages for a first predetermined block of the intervals;
generating prospective averages for a second predetermined block of the intervals, where the second block has an overlap with the first block;
generating combined averages for the intervals relating to the overlap, the combined averages being derived from the retrospective averages and the prospective averages, with one such combined average relating to each of the predetermined intervals; and
substituting the retrospective averages, the prospective averages, and the combined averages for the flow readings in the memory.

17. The method of claim 16, wherein:
each of the retrospective averages is generated by first generating a retrospective total of the flow readings from the first predetermined interval through the Ith predetermined interval, where I is an integer between 2 and a first predetermined constant J, and then dividing the retrospective total by I, and repeating the step of generating such retrospective averages for each I between 2 and J;
each of the prospective averages for the intervals between (J-K) and (N-K) is generated by first generating a prospective total of the flow readings from the Lth predetermined interval through the (L+K)th predetermined interval, where K is a second predetermined constant and L is an integer between (J-K) and N-K, where N is the total number of predetermined intervals, and then dividing the prospective total by K+1, and repeating the step of generating such prospective averages for each L between (J-K) and (N-K);
each of the combined averages for the respective predetermined intervals is generated by adding a fraction (J-M)/(K+1) of the retrospective average for the predetermined interval to a fraction (M-(J-(K+1)))/(K+1) of the prospective average for the predetermined interval, where M is the number of the interval for which the combined average is being generated, and carrying out the step of generating such combined averages for each predetermined interval between (J-K) and J; and wherein
the step of substituting the retrospective averages, the prospective averages and the combined averages for the flow readings in the memory includes substituting the flow readings in the memory with the retrospective averages for the intervals between 1 and (J-(K+1)), substituting the volume readings in the memory with the combined averages between the intervals (J-K) and J, and substituting the volume readings in the memory with the prospective averages between the intervals (J + 1) and (N-K).

18. The method of claim 15, wherein the sampling of the capacitance values is carried out at a predetermined clock rate, and further including the steps of:

generating a total fluid flow time value by determining the number of the predetermined intervals during which fluid flow into the vessel was taking place, and by dividing this number of predetermined intervals by the clock rate; and displaying the total fluid flow time value.

19. A method for determining flow rates of a fluid into a vessel having an interior for collecting a fluid, wherein a variable capacitor in communication with the interior has varying capacitance values with the capacitance value at a given time being a function of the amount of fluid in the vessel at that time, including the steps of:

sampling the capacitance values at predetermined intervals for obtaining volume readings having values reflecting the amount of fluid in the interior of the vessel at each such interval;

comparing volume readings obtained at succeeding predetermined intervals for generating flow readings from the differences between successive volume readings;

generating a total fluid volume value by taking a difference between the volume readings for a first such predetermined interval and a last such predetermined interval; and

displaying the flow readings and the total fluid volume value.

20. The method of claim 19, further including the steps of:

generating an average fluid flow value by taking an average of all of the flow readings;

generating a peak fluid flow value by comparing flow readings for succeeding predetermined intervals, determining the highest such flow reading, and taking an average of the flow readings for five such predetermined intervals around and including the highest flow reading; and

displaying the average fluid flow value and the peak fluid flow value.

**Fig. 1**

**Fig. 2**

Fig. 3

Fig. 4

**Fig. 5**

**Fig. 6**

**Fig. 7**

VARIABLE CAPACITOR — 220

SINE WAVE OSCILLATOR — 350

CAPACITANCE BRIDGE — 340

REFERENCE GENERATOR — 430

AC GAIN — 400

BAND PASS FILTER — 410

SYNCHRONOUS DEMODULATOR — 420

DC GAIN — 440

LOW PASS FILTER — 450

VOLTAGE-TO-FREQUENCY CONVERTER — 460

MICROPROCESSOR — 470

EPROM — 480

RAM — 490

INTERFACE CIRCUITS — 510

PRINTER — 520

CONTROL PANEL — 500

**Fig. 8**

**Fig. 9**

**Fig. 10**

START MEASURING LOOP

OBTAIN CURRENT VOLUME COUNT FOR
1/5 SECOND INTERVALS FOR 90 SECONDS

CURRENT VOLUME COUNT LESS THAN
PREVIOUS VOLUME COUNT?

YES

NO

SET CURRENT
DIFFERENCE =
PREVIOUS
DIFFERENCE

**Fig. 11**

CALCULATE CALIBRATION
DATA FOR PRINTER

IMPLEMENT VESSEL
CORRECTION FACTOR

STORE DATE IN RAM

EXIT LOOP

```
┌─────────────────────────────────────┐
│     START  POST-PROCESSING  LOOP     │
└─────────────────────────────────────┘
                    │
                    ▼
┌─────────────────────────────────────┐
│       TAKE  TOTAL  AVERAGE  OF       │
│    COUNTS  AT  EACH  INTERVAL        │
│        UP· TO  16th  INTERVAL        │
└─────────────────────────────────────┘
                    │
                    ▼
┌─────────────────────────────────────┐
│   GENERATE  8-POINT  PROSPECTIVE     │
│    AVERAGES  FOR  17th  INTERVAL     │
│          TO  END  OF  DATA           │
└─────────────────────────────────────┘
                    │
                    ▼
┌─────────────────────────────────────┐
│        BEGIN  DATA  MERGING          │
└─────────────────────────────────────┘
                    │
                    ▼
┌─────────────────────────────────────┐
│   FROM t = 0  TO  16th INTERVAL:  USE│
│     RETROSPECTIVE  AVERAGES  ONLY    │
└─────────────────────────────────────┘
                    │
                    ▼
```

## Fig. 12

```
┌───────────────────────────────────────────────────────┐
│  FROM  17th  INTERVAL  TO  24TH  INTERVAL:            │
│                                                       │
│  LET COUNT = [1/8 x (24 - NO. OF DATA INTERVAL)       │
│                      X RETROSPECTIVE AVERAGE]          │
│                          +                            │
│              [1/8 x (NO. OF DATA INTERVAL - 16)       │
│                      X PROSPECTIVE AVERAGE]            │
└───────────────────────────────────────────────────────┘
                    │
                    ▼
┌─────────────────────────────────────┐
│  FROM 25th INTERVAL TO END OF DATA:  │
│   USE  PROSPECTIVE  AVERAGES  ONLY   │
└─────────────────────────────────────┘
                    │
                    ▼
┌─────────────────────────────────────┐
│     EXIT  POST-PROCESSING  LOOP      │
└─────────────────────────────────────┘
```

# Fig. 13

# Fig. 14